# EUROPEAN PATENT APPLICATION

(11) **EP 4 489 021 A1**
(43) Date of publication of application: **08.01.2025**
(21) Application number: 21962556.3
(22) Date of filing: 27.10.2021
(51) Int. Cl.: G16H 50/30, G16H 50/20, G16H 80/00, A61B 5/00, A61B 5/145

(54) **BODY FLUID MANAGEMENT SERVER AND METHOD**

(30) Priority: 26.10.2021 KR 20210144050
(71) Applicant: INTIN INC., Daegu 41069 (KR)
(72) Inventor: KIM, Ji Hoon, Seoul 18449 (KR)
(74) Representative: RGTH
(86) International application number: PCT/KR2021/015194
(87) International publication number: WO 2023/074930

(57) **Abstract**

The present invention relates to a body fluid management server and a body fluid management method using the server, the server comprising: an interface unit for performing communication interface between user equipment, which is communication-connected to a personal sperm measurement apparatus, and a clinic sperm measurement apparatus; a body fluid analysis unit for acquiring the state information of sperm by analyzing clinic sperm data and personal sperm data received via the interface unit, and providing the state information with respect to the personal sperm data to the user equipment and the clinic sperm measurement apparatus or a clinic terminal to enable monitoring by a user and a clinic authorized person; a body fluid state determination unit for determining the final state of the sperm of the relevant user by means of the state information of the sperm acquired by the body fluid analysis unit; a health management information generation unit for generating health management information suitable for the user in accordance with the level of the management state from among the final state determined by the body fluid state determination unit, and providing same to the user equipment; a measurement data storage unit for storing clinic sperm data and personal sperm data of respective users; and health management information storage unit for storing health management information provided to respective users.

## Description

### [TECHNICAL FIELD]

The present disclosure relates to a technology for managing measurement data and improvement history of body fluids such as sperm on the Internet, and more particularly, to a body fluid management server and a method for providing a user with personalized user management services using measurement data from a hospital-grade body fluid analyzer and a personal-grade body fluid analyzer.

### [BACKGROUND ART]

Sperm, among the body fluids in a man's body, plays a crucial role, and hospitals are organizing fertility clinics specifically aimed at individuals with poor sperm quality. Understanding sperm state is vital for men and couples alike, and there is a growing interest among men in assessing the state of sperm, as well as among husbands facing challenges with delayed or unsuccessful conception.

In order to assess the state of sperm, clinics are equipped with clinic-grade fluid testers, that is, sperm testers, and to measure the state of each individual's sperm using the clinic-grade sperm testers. In addition, there are personal-grade sperm testers possible, which, though less functional than clinic-grade ones, are more affordable.

In general, fertility clinics prescribe medications and recommend diet and exercise if the state of sperm is not good according to a result of sperm measurement, and a patient may be asked to visit the clinic periodically, such as every month or two, to see if the state of sperm has improved.

However, this method used by fertility clinics only assesses the sperm state during the patient's clinic visits, making it impossible to frequently monitor changes in sperm state and provide immediate prescriptions.

### [DETAILED DESCRIPTION OF INVENTION]

### [TECHNICAL PROBLEMS]

The present disclosure provides a body fluid management server and method for managing sperm data measured at a clinic and sperm data measured by a user and providing health management information, which is an enhancement program that enables individuals to improve sperm state based on the measured sperm data.

In addition, the present disclosure provides a body fluid management server and method for identifying changes in sperm state based on sperm data measured by an individual after receiving health management information, thereby enabling a clinic administrator to immediately assess the individual's adherence to the provided health management information, ensuring continuous management.

In addition, the present disclosure provides a body fluid management server and method for allow sperm data measured at a clinic, sperm data measured by an individual, and important information to be shared in an encrypted form with blockchain nodes on a blockchain network.

In addition, the present disclosure provides a body fluid management server and method for providing each individual's sperm measurement data meeting a condition requested by an external organization or company.

### [TECHNICAL SOLUTION]

In order to address the above problems, a body fluid management server according to one aspect of the present disclosure includes: an interface unit configured to perform communication interface with a clinic terminal in communication with a clinic sperm measurement apparatus and a user terminal in communication with a personal sperm measurement apparatus; a body fluid analysis unit configured to assess state information of sperm by analyzing clinic sperm data and personal sperm data received via the interface unit, and provide state information regarding the personal sperm data to the user terminal and the clinic terminal for monitoring by the user and a clinic administrator; a body fluid state determination unit configured to determine a final state of the sperm of the user based on the state information of the sperm assessed by the body fluid analysis unit; a health management information generation unit configured to generate health management information suitable for the user in accordance with a level of a care state from among the final state determined by the body fluid state determination unit; a measurement data storage unit configured to store each user's clinic sperm data and personal sperm data; and a health management information storage unit configured to store the health management information provided to each user.

The body fluid analysis unit may analyze each of a plurality of measurement images included in the clinic sperm data to assess state information of sperm in the respective measurement images, and assess final state information by averaging the determined state information of sperm in the respective measurement images.

When the interface unit receives biometric information of the user from the user terminal, the body fluid state determination unit may determine a final state based on the sperm state information assessed by the body fluid analysis unit and the biometric information of the user.

According to one aspect of the present disclosure, the body fluid management server may further include a blockchain unit configured to generate a block by encrypting the clinic sperm data and the personal sperm data and transmit the generated block to each blockchain node of a blockchain associated with the user to share information.

According to one aspect of the present disclosure, the body fluid management server may further include: a user information assessment unit configured to inquire the user about personal information regarding at least one of an existing disease, a dietary habit, an alcohol consumption status, and a smoking status, receive a response from the user, and assess the user's characteristics based on the received personal information; and a selection information provision unit configured to, in response to receiving a request from an external organization or company for sperm data exhibiting a specific individual's characteristics, retrieve the data exhibiting the specific individual's characteristics based on the characteristics assessed by the user information assessment unit and provide the retrieved data to the external organization or company.

According to one aspect of the present disclosure, the body fluid management server may further include a reward provision unit configured to provide a reward to a user who has provided the personal sperm data or a user who owns the sperm data provided to the external organization or company through the selective information provision unit.

According to one aspect of the present disclosure, the body fluid management server may further include an insurance linkage unit configured to suggest insurance enrollment or claim to a user when the sperm state determined by the body fluid state determination unit has changed from the care state to the normal state, from the care state to the abnormal state, or when a level of the care state decreases or increases to a set level, and provide an insurance company with information on the user's sperm state when receiving a positive response from the user.

In order to address the above problems, a body fluid management server according to another aspect of the present disclosure includes: an interface unit configured to perform communication interface with a clinic terminal in communication with a clinic sperm measurement apparatus and a user terminal in communication with a personal sperm measurement apparatus; a data identification unit configured to identify a type of data received through the interface unit and a user for the data, and provide the data to the clinic terminal when the received data is personal sperm data; a body fluid state determination unit configured to store the clinic sperm data and personal sperm data received through the interface unit, and analyze state information of sperm received from the clinic terminal through the interface unit to assess a final sperm state of a user; a health management information generation unit configured to provide a user whose sperm is in a care state with health management information based on an analysis result from the state information analysis unit; a measurement data storage unit configured to store each user's clinic sperm data and personal sperm data; a health management information storage unit configured to store health management information provided to each user; and a state information storage unit configured to store the state information of sperm, transmitted from the clinic terminal.

In order to address the above problems, a health management method according to one aspect of the present disclosure is performed through a body fluid management server which is connected to a user terminal in communication with a personal sperm measurement apparatus and a clinic terminal in communication with a clinic sperm measurement apparatus to manage a health status of sperm, and the method includes: analyzing, by the body fluid management server, received clinic sperm data to assess first state information of sperm; assessing, by the body fluid management server, a final state of a user's sperm based on the first state information; providing, by the body fluid management server, health management information corresponding to the final state to the user terminal and the clinic terminal; analyzing, by the body fluid management server, received personal sperm data to assess second state information of the sperm; and providing, by the body fluid management server, the second state information to the user terminal and the clinic terminal so that the user and a clinic administrator monitor the second state information.

The assessing of the first state information may include analyzing each of a plurality of measurement images included in the clinic sperm data to assess state information of sperm in the respective measurement images, and assessing the first state information by averaging the assessed state information of sperm in the respective measurement images.

The assessing of the final state comprises determining the final state based on the first state information and biometric information of the user when the biometric information of the user is received from the user terminal.

The body fluid management method according to one feature of the present disclosure further comprises generating a block by encrypting the clinic sperm data and the personal sperm data and transmitting the generated block to each blockchain node of a blockchain associated with the user to share information..

The body fluid management method according to one aspect of the present disclosure may further include: inquiring the user about personal information regarding at least one of an existing disease, a dietary habit, an alcohol consumption status, and a smoking status, receiving a response from the user, and assessing the user's characteristics based on received personal information; and in response to receiving a request from an external organization or company for sperm data exhibiting a specific individual's characteristics, retrieving the data exhibiting the specific individual's characteristics and providing the retrieved data to the external organization or company.

The body fluid management method according to one feature of the present disclosure may further include providing a reward to the user who provided the personal sperm data or to the user who is the owner of the sperm data provided to an external organization or company.

The body fluid management method according to one aspect of the present disclosure may further include suggesting insurance enrollment or insurance claim to a user when the sperm state determined by the body fluid state determination unit has changed from a care state to a normal state, from the care state to an abnormal state, or when a level of the care state decreases or increases to a set level, and providing an insurance company with information on the user's sperm state when receiving a positive response from the user.

In order to address the above problems, a health management method according to another aspect of the present disclosure is performed through a body fluid management server which is connected to a user terminal in communication with a personal sperm measurement apparatus and a clinic terminal in communication with a clinic sperm measurement apparatus to manage health state of sperm, and the method includes: storing, by the body fluid management server, received clinic sperm data or received personal sperm data; analyzing, by the body fluid management server, sperm state information regarding the received clinic sperm data to assess the final state of a user's sperm; providing, by the body fluid management server, health management information corresponding to the final state to the user terminal and the clinic terminal; and in response to receiving personal sperm data, providing, by the body fluid management server, the personal sperm data to the clinic terminal so that clinic administrator monitor the personal sperm data.

### [EFFECT OF INVENTION]

In the present disclosure, it is possible to provide a personalized management service suitable for a current situation based on sperm data measured by a clinic body fluid measurement apparatus or a personal body fluid measurement apparatus at any given time.

In addition, in the present disclosure, it is possible to provide sperm data for a specific region, age group, specific illness, or specific condition requested by an external organization or company for clinical or experimental purposes.

In addition, in the present disclosure, it is possible to provide evidence of the reliability of an individual's current sperm state, enabling the user to subscribe to infertility-related insurance or claim insurance premiums.

### [BRIEF DESCRIPTION OF THE DRAWING]

FIG. 1 is a diagram showing a network environment of a body fluid management server according to an embodiment of the present disclosure.
FIG. 2 is a block diagram of a body fluid management server according to a first embodiment of the present disclosure.
FIG. 3 is a data flowchart for a body fluid management method according to the first embodiment of the present disclosure.
FIG. 4 is a data flowchart for a body fluid management method according to a second embodiment of the present disclosure.
FIG. 5 is a block diagram of a body fluid management server according to the second embodiment of the present disclosure.
FIG. 6 is a diagram showing information sharing over a blockchain network according to an embodiment of the present disclosure.
FIG. 7 is a block diagram of a body fluid management server according to a third embodiment of the present disclosure.
FIG. 8 is a data flowchart for a body fluid management method according to a third embodiment of the present disclosure.
FIG. 9 is a data flowchart for an insurance company linking operation according to an embodiment of the present disclosure.
FIG. 10 is a block diagram of a body fluid management server according to a fourth embodiment of the present disclosure.
FIG. 11 is a data flowchart for a body fluid management method according to a fifth embodiment of the present disclosure.
FIG. 12 is a diagram showing personal sperm data and clinic sperm data according to an embodiment of the present disclosure.
FIG. 13 is a diagram showing a screen of a service application installed on a user terminal according to an embodiment of the present disclosure.
FIG. 14 is a diagram showing an example of health management information according to an embodiment of the present disclosure.
FIG. 15 is a diagram showing the contents of a questionnaire provided through a service application according to an embodiment of the present disclosure.

### [MODE FOR CARRYING OUT THE INVENTION]

Description will now be given in detail according to exemplary embodiments disclosed herein, with reference to the accompanying drawings, and the same or equivalent components may be provided with the same or similar reference numbers, and description thereof will not be repeated. In addition, in the following description of the embodiments, a detailed description of known functions and configurations incorporated herein will be omitted when it may impede the understanding of the embodiments.

While terms including ordinal numbers, such as "first" and "second," etc., may be used to describe various components, such components are not limited by the above terms. The above terms are used only to distinguish one component from another.

The singular forms "a," "an" and "the" are intended to include the plural forms as well, unless the context clearly indicates otherwise.

In this application, each step described may be performed regardless of the listed order, except when it must be performed in the listed order due to a special causal relationship.

It will be further understood that the terms "comprise", "include", "have", etc. when used in this specification, specify the presence of stated features, integers, steps, operations, elements, components, and/or combinations of them but do not preclude the presence or addition of one or more other features, integers, steps, operations, elements, components, and/or combinations thereof.

Hereinafter, the present disclosure will be described with reference to the attached drawings.

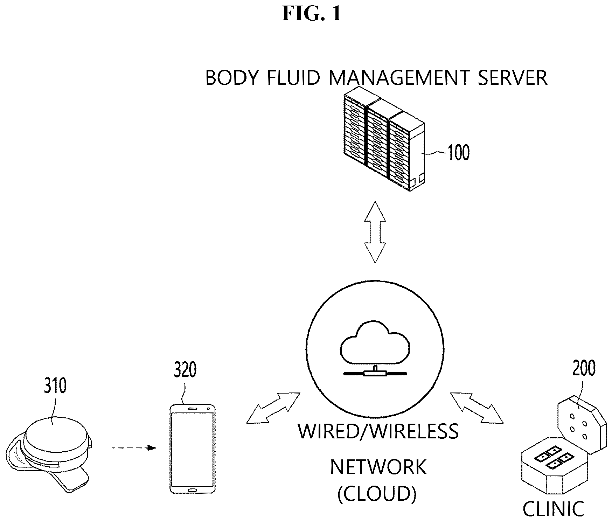

FIG. 1 is a diagram showing a network environment of a body fluid management server according to an embodiment of the present disclosure. Referring to FIG. 1, a body fluid management server 100 according to an embodiment of the present disclosure is connected to a wired or wireless network (including the cloud), and includes a clinic sperm measurement apparatus 200 and user terminal 320 connected to the wired or wireless network. Here, the clinic sperm measurement apparatus 200 may be connected to the clinic terminal by wire or wirelessly, and the clinic terminal may be connected to the body fluid management server 100 over a network, or the clinic sperm measurement apparatus 200 and a clinic terminal may be integrated.

The body fluid management server 100 according to an embodiment of the present disclosure receives clinic sperm data provided by the clinic sperm measurement apparatus 200 and personal sperm data provided by the user terminal 320 and provides a personalized management service.

For example, the personalized management service is a health management information program (hereinafter referred to as "health management information") for improving the quality of sperm based on the user's clinic sperm data and/or personalized sperm data, as well as a monitoring service regarding regularly received personal sperm data.

The health management information includes information regarding at least one of food and health, and is recommended information regarding at least one of food and health, the information provided daily, every other day, or weekly during a set period. Of course, the health management information may be configured for any timeframe determined by the user or clinic administrator, and may be configured based on the user's hospital visit frequency.

In addition, the personalized management service may further include at least one of the following: an information-sharing service for encrypting users' clinic sperm data and/or personal sperm data and sharing the data through blockchain nodes of relevant parties connected to a blockchain network; a reward provision service for providing specific-condition sperm data in response to an external request and rewards the owner of the provided sperm data; and an insurance support service for supporting infertility-related insurance.

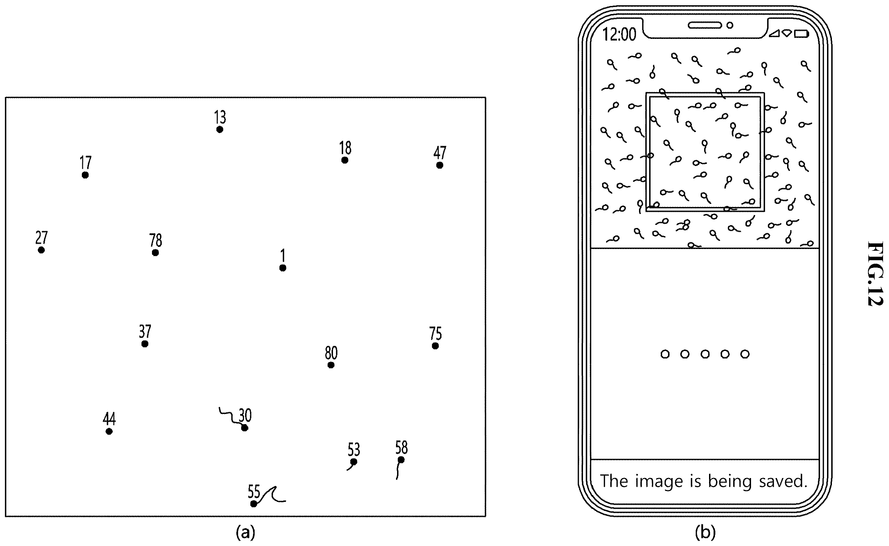

The clinic sperm measurement apparatus 200 has a plurality of chambers, and simultaneously measures the state of sperm in each of the respective chambers and provides results of measurement, that is, multiple clinic sperm data, to the body fluid management server 100. Clinic sperm data is provided in the form of multiple images or videos, which, when viewed as still images, may be shown as in (a) of FIG. 12. Because the clinic sperm measurement apparatus 200 uses a high-magnification lenses to measure the state of sperm, the clinic sperm measurement apparatus 200 is a high-performance and expensive device compared to a personal sperm measurement apparatus 310.

The clinic sperm measurement apparatus 200 may be directly connected to the body fluid management server 100, but may also be connected to the body fluid management server 100 through a clinic terminal (not shown). The clinic terminal is a device that enables the user to visually check clinic sperm data measured by the clinic sperm measurement apparatus 200, and may have a function of assessing state information of sperm. The state information of sperm includes the number of sperm, sperm motility, sperm viability, sperm morphology, etc.

The user terminal 320 may be a smartphone or a computer, and is connected to the personal sperm measurement apparatus 310 by wire or wirelessly, and the personal sperm data measured by the personal sperm measurement apparatus 310 may be provided in the form of multiple images or videos, which, when viewed as still images, may be shown as in (b) of FIG. 12.

The body fluid management server 100 provides a service application to the user terminal 320 of the smartphone to provide the service, and the user may be provided with services by executing the service application and then connecting the body fluid management server 100 through the service app.

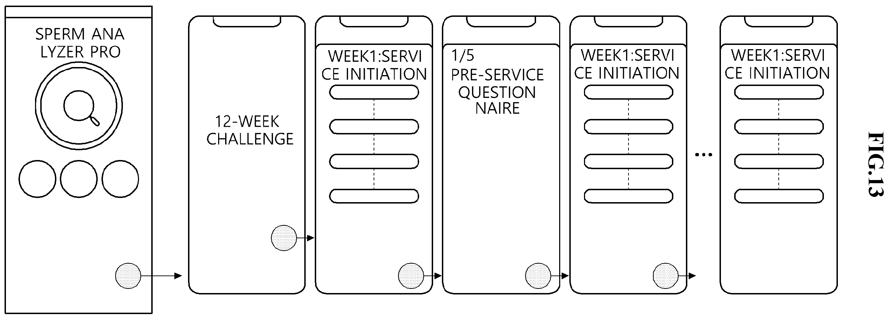

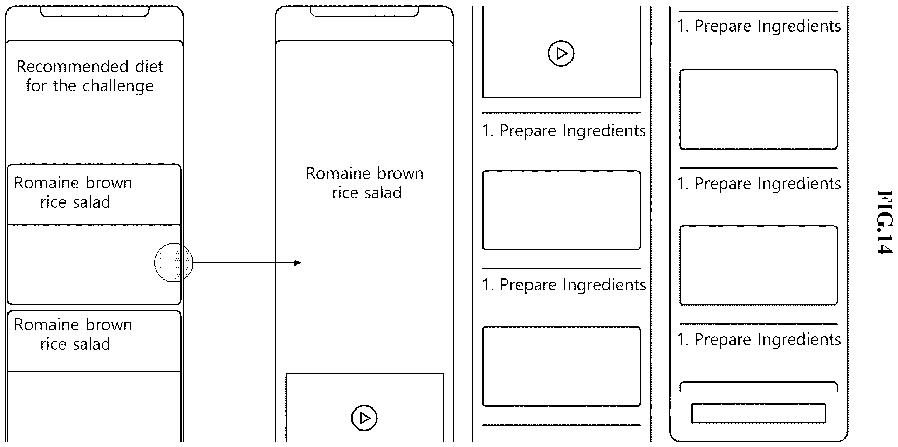

An example of the service application running on the user terminal 320 of the smartphone is shown in FIGS. 13 and 14. FIG. 13 is a diagram showing a screen of a service application installed on a user terminal according to an embodiment of the present disclosure, and FIG. 14 is a diagram showing an example of health management information according to an embodiment of the present disclosure.

Referring to FIGS. 13 and 14, the service application is connected to the personal sperm measurement apparatus and provides a screen for signing up to access the services. Furthermore, the service application provides the user with health management information, recommending the consumption of foods that can improve sperm quality, such as 'Romaine Brown Rice Salad,' and offers a screen displaying the recipe for 'Romaine Brown Rice Salad.'

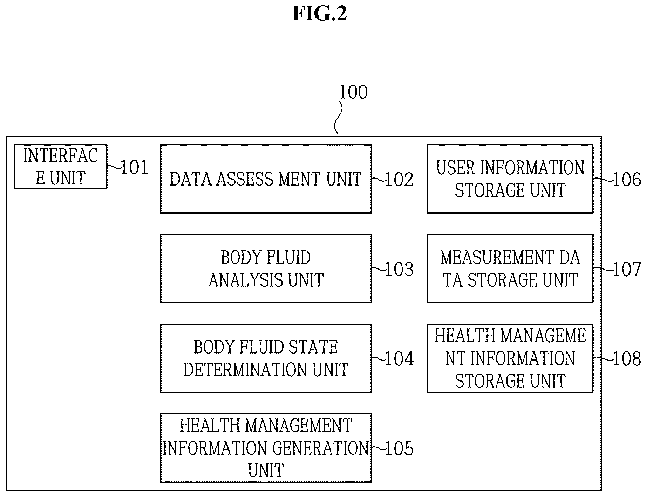

FIG. 2 is a block diagram of a body fluid management server according to a first embodiment of the present disclosure. Referring to FIG. 2, a body fluid management server 100 according to the first embodiment of the present disclosure includes an interface unit 101, a data identification unit 102, a body fluid analysis unit 103, a body fluid state determination unit 104, a health management information generation unit 105, a user information storage unit 106, a measurement data storage unit 107, and a health management data storage unit 108.

The interface unit 101 performs a communication interface with an external terminal connected from the outside to enable data transmission and reception with respect to the external terminal. The data identification unit 102 identifies the type of data received through the interface unit 101 and identifies the sender, i.e., the user, by assessing source information of the data.

The body fluid analysis unit 103 analyzes received clinic sperm data and personal sperm data to assess state information of sperm. For example, in the case of the clinic sperm data, since the clinic sperm data consists of multiple measurement images, the body fluid analysis unit 103 assesses the state information of sperm in the respective measurement images and assesses final state information of sperm by averaging assessed state information of sperm in the respective measurement image. In the case of the personal sperm data, the body fluid analysis unit 103 provides the assessed state to the user terminal 320 and the clinic side (i.e., the clinic sperm measurement apparatus and/or hospital terminal).

The body fluid state determination unit 104 determines the final state of the user's sperm based on an analysis result from the body fluid analysis unit 103. The state of sperm includes a normal state where the sperm condition is normal, an abnormal state where the sperm condition is not under care, and a care state where the sperm condition can improve with care, offering multiple levels of the care state based on the degree of sperm condition. When the final state of the personal sperm data is determined, the fluid state determination section 104 may provide the final state to the user terminal 320 and the clinic side (i.e., the clinic sperm measurement apparatus and/or the clinic terminal).

The health management information generation unit 105 generates health management information (i.e., sperm enhancement program) for a user with the care state of sperm, and at this point, health management information suitable for the user is generated based on the level of the care state. For example, higher levels of the care state involve providing management information that emphasizes increased exercise intensity and duration for the user, along with recommending foods known to enhance sperm condition. And, when receiving personal sperm data from the user after providing the health management information to the user, the health management information generation unit 105 may provide new or one-time health management information depending on the state of the received personal sperm data.

A higher level of the care state implies that the sperm's state is poorer compared to a lower level of the care state.

The user information storage unit 106 stores personal information (e.g., name, age, residence, weight, height, etc.) for registered users. The measurement data storage unit 107 stores each user's clinic sperm data and personal sperm data. The health management information storage unit 108 stores health management information provided for each user.

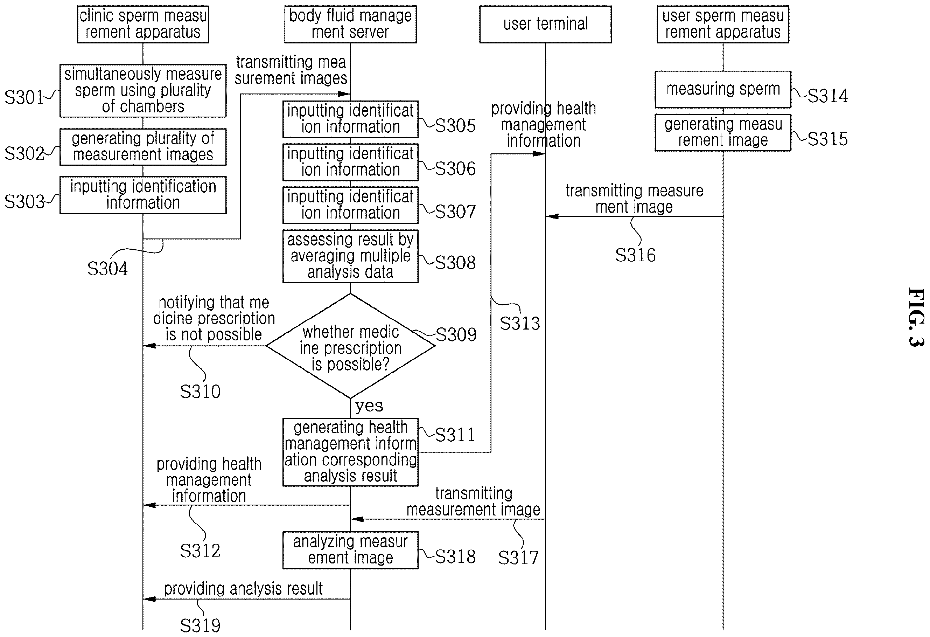

FIG. 3 is a data flowchart for a body fluid management method according to the first embodiment of the present disclosure, showing operations of the body fluid management server 100 according to the first embodiment of the present disclosure.

Referring to FIG. 3, when a user visits a clinic, the measurement is initiated with the user's sperm placed into a plurality of chambers of a clinic sperm measurement apparatus 200. Subsequently, the clinic sperm measurement apparatus 200 simultaneously measures the user's sperm in the plurality of chambers (S301), and generates a plurality of measurement images for sperm in the respective chambers (S302). The clinic sperm measurement apparatus 200 adds the user's identification information (e.g., name or code number corresponding to the name, etc.) to a message for the plurality of generated measurement images (S303), and transmits a measurement image message, containing the plurality of measurement images and the user's identification information, to the body fluid management server 100 (S304).

The body fluid management server 100 receives the measurement image message (S305), identifies the user based on the user's identification information contained in the message, and stores the measurement images matched with the user in the measurement data storage unit 106 (S306).

Next, the body fluid management server 100 performs image analysis on each of the user's multiple measurement images to assess state information of sperm, such as the number of sperm, sperm motility, sperm viability, sperm morphology, etc. (S307). Next, the body fluid management server 100 assesses final state information for the user's sperm by averaging the assessed states of sperm (S308).

The body fluid management server 100 compares the final state information with setting information to determine whether the user's sperm is in a normal, abnormal, or care state and whether medicine prescription is possible, and determines what level of the care state in response to a determination that the user's sperm is in the care state (S309).

When the sperm is in the normal or abnormal state, the body fluid management server 100 notifies both the clinic sperm measurement apparatus 200 and a user terminal 320 and stops operation (S312). On the other hand, when the sperm is in the care state, the body fluid management server 100 generates health management information corresponding to an analysis result (S311), provides the generated health management information to both the user terminal 320 and the clinic sperm measurement apparatus 200, informing the user and a clinic administrator (S312, S313). Of course, even if the user's sperm is the care state, the body fluid management server 100 also notifies the user terminal 320 and the clinic sperm measurement apparatus 200 of the care state.

When the user checks the health management information through the user terminal 320, the user follows the schedule included in the health management information to improve the sperm state by consuming specific foods or performing exercises. Next, the user measures his sperm state using a personal sperm measurement apparatus 310 in order to assess the impact of the user's actions in accordance with the health management information.

Next, the personal sperm measurement apparatus 310 measures the state of the sperm (S314), generates a measurement image (S315), and provides the generated measurement image to the user terminal 320 (S316).

The user terminal 320 receives the measurement images from the personal sperm measurement device 310, displays the received measurement images on the screen of the user terminal 320 through a service application for user confirmation, and transmits the measurement images to the body fluid management server 100 as per the user's instruction (S317).

When receiving the measurement images from the user terminal 320, the body fluid management server 100 identifies the user, stores the received measurement images matched with the user, and then analyzes the measurement images measured by the personal sperm measurement apparatus 310 to assess state information of sperm (S318).

The body fluid management server 100 provides the state information of the user's sperm assessed in operation S318 to the clinic measurement apparatus 200 (S319). Of course, the body fluid management server 100 may provide the user terminal 310 with the state information of the user's sperm assessed in operation S318.

Therefore, the clinic administrator is able to monitor whether the user is following the health management information by receiving and verifying the user's sperm state, measured through the personal sperm measurement apparatus 310, via the body fluid management server 100.

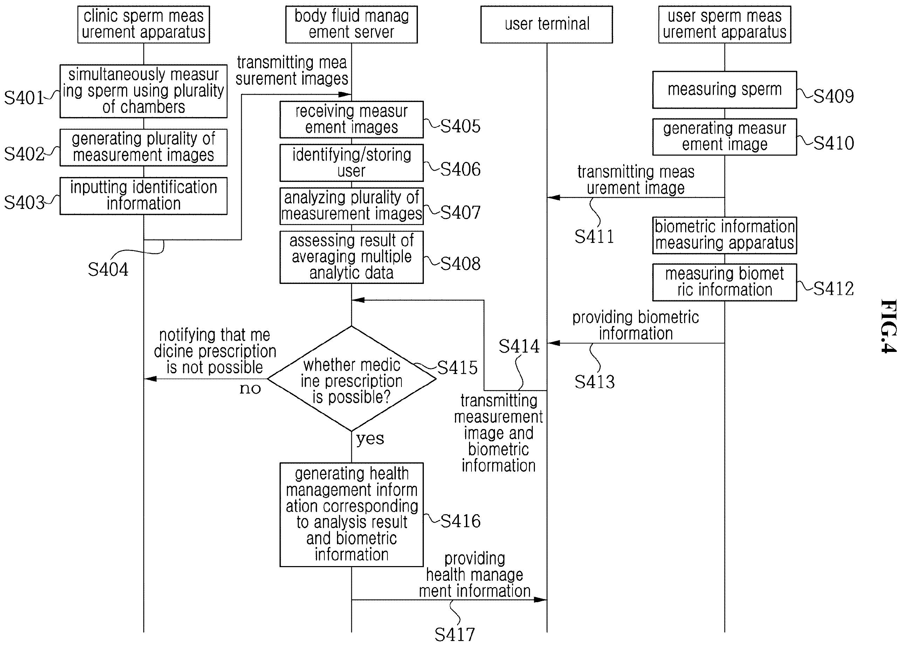

FIG. 4 is a data flowchart for a body fluid management method according to a second embodiment of the present disclosure. The body fluid management method according to the second embodiment of the present invention is generally similar to the first embodiment of the present disclosure, but differs in that the user's sperm state assessment and health management information generation are performed using measurement images obtained from a personal sperm measurement apparatus 310 and the user's biometric information.

To explain this more specifically, a clinic sperm measurement apparatus 200 performs the same operations as operations S301 to S304 and transmits the user's clinic measurement images to a body fluid management server 100 (S401 to S404), and the body fluid management server 100 performs the same operation as operations S305 to S308 to assess an analysis result of the user's sperm (S405 to S408).

In this state, the user performs sperm measurement using the personal sperm measurement apparatus 310, and the personal sperm measurement apparatus 310 measures the user's sperm (S409), generates a measurement image for the user's sperm state (S410), and provides the measurement image to the user terminal 320 (S411).

Next, the user measures biometric information through a biometric information measurement apparatus (S412), and the biometric information measurement apparatus provides the measured biometric information to the user terminal 320 (S413). The biometric information includes at least one of BMI (Body Mass Index), heart rate, body composition, etc.

The user terminal 320 provides the body fluid management server 100 with the biometric information measured using the personal sperm measurement apparatus 310 and the measurement image measured using the personal sperm measurement apparatus 310 (S414).

When receiving the measurement image from the user terminal 320, the body fluid management server 100 identifies the user, generates health management information based on an analysis result for the measurement image measured by the personal sperm measurement apparatus 310 and the received biometric information of the user, in addition to the analysis result assessed in operation S408 (S416), and provides the user terminal 320 with the generated health management information (S417).

Of course, the body fluid management server 100 also provides the health management information generated in operation S416 to the clinic sperm measurement apparatus 200.

After providing the health management information to the user terminal 310, the body fluid management server 100 receives the measurement images measured by the personal sperm measurement apparatus 310 and transmitted through the same operations as operations S314 to S316, and provides an analysis result for the measurement image measured by the personal sperm measurement apparatus 310 to both the clinic sperm measurement apparatus 300 and the user terminal 320 through the same operations as operations S318 to S319.

Meanwhile, after the body fluid management server 100 provides the health management information to the user terminal 310, the user may measure the sperm using the personal sperm measurement apparatus 310 and measure the biometric information, and then provide a result to the body fluid management server 100 through the user terminal 320. In this case, if there is a significant change in the state of the sperm measured by the personal sperm measurement apparatus 310, such as a decrease or increase in the level of the care state compared to the previous level, the body fluid management server 100 may generate new health management information and provide the generated new health management information to the clinic and the user. In addition, if the received biometric information shows a change of a predetermined magnitude compared to previously received biometric information, the body fluid management server 100 may generate new health management information and provide the generated new health management information to the clinic and the user.

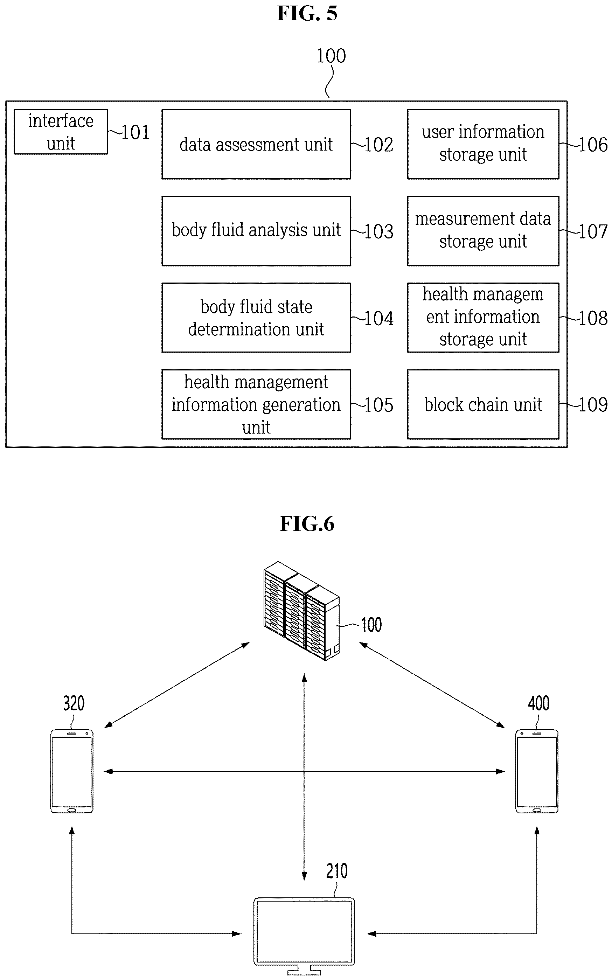

FIG. 5 is a block diagram of a body fluid management server according to a second embodiment of the present disclosure, and FIG. 6 is a diagram showing information sharing using a blockchain network according to an embodiment of the present disclosure.

Referring to FIGS. 5 and 6, a body fluid management server 100 according to the second embodiment of the present disclosure includes an interface unit 101, a data identification unit 102, a body fluid analysis unit 103, a body fluid state determination unit 104, a health management information generation unit 105, a user information storage unit 106, a measurement data storage unit 107, a health management information storage unit 108, and a blockchain unit 109.

The body fluid management server 100 according to the second embodiment of the present disclosure retains the same configuration as the body fluid management server 100 according to the first embodiment of the present disclosure, with the inclusion of the blockchain unit 109.

The blockchain unit 109 generate a block by encrypting a measurement image received from a clinic sperm measurement apparatus 200, a measurement image received from the personal sperm measurement apparatus 200, and information (e.g., health management information, etc.) transmitted by the body fluid management server 100 to a user terminal 320 and the clinic sperm measurement apparatus 200, and transmits the generated block to blockchain nodes associated with the user for sharing information. For example, the blockchain nodes include the body fluid management server 100, the user terminal 320, a clinic administrator terminal 400, a clinic terminal 210 (or a clinic sperm measurement apparatus), etc.

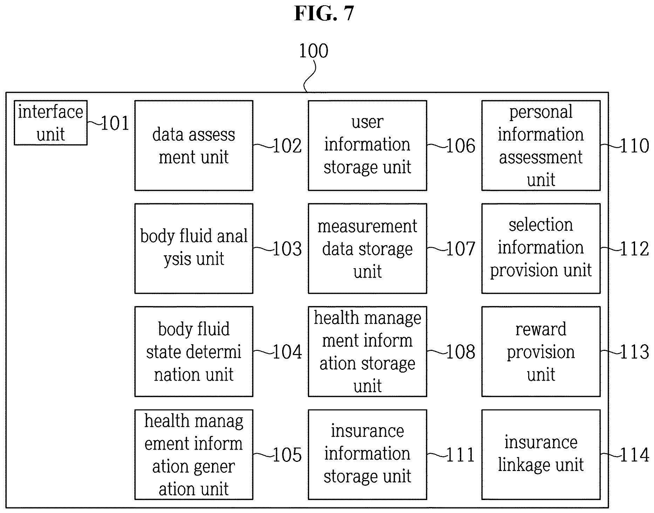

FIG. 7 is a block diagram of a body fluid management server according to a third embodiment of the present disclosure. Referring to FIG. 7, a body fluid management server 100 according to the third embodiment of the present disclosure includes an interface unit 101, a data identification unit 102, a body fluid analysis unit 103, a body fluid state determination unit 104, a health management information generation unit 105, a user information storage unit 106, a measurement data storage unit 107, a health management information storage unit 108, a personal information assessment unit 110, an insurance information storage unit 111, a selection information provision unit 112, a reward provision unit 113, and an insurance linkage unit 114. Meanwhile, the body fluid management server 100 according to the third embodiment of the present disclosure may further include the aforementioned blockchain unit 109.

The interface unit 101, data identification unit 102, body fluid analysis unit 103, body fluid state determination unit104, health management information generation unit 105, measurement data storage unit 107, and health management information storage unit 108 of the body fluid management server 100 according to the third embodiment of the present disclosure are the same as those described in the first and second embodiments of the present disclosure, so detailed descriptions thereof are omitted.

The personal information assessment unit 110 inquires a user about personal information, receives a response from the user, and stores the received personal information matched with user identification information in the user information storage unit 106. Here, the personal information includes the user's physical condition (e.g., existing diseases, allergy symptoms, etc.), dietary habits, alcohol consumption status, smoking status, etc.

The insurance information storage unit 111 stores information on infertility-related insurance that each user has been enrolled in.

When receiving a request for sperm data (i.e., sperm measurement images for clinic or personal use) meeting a specific condition from an external organization or company, the selection information provision unit 112 retrieves sperm data meeting the specified condition from the measurement data storage unit 106 and provides the sperm data to the corresponding external organization or company. In this case, the specific condition is a combination of at least one of the following: a specific location (city, county, district, town, village, neighborhood, etc.), a specific age, a specific illness (e.g., diabetes, hypertension, allergies, heart disease, kidney disease, etc.), gender, and whether a sperm condition has deteriorated (or improved) during a set period.

The reward provision unit 113 provides a reward to the user who meets the set condition. The provided reward is stored in the user information storage unit 106, matched with the user's identification information. Here, the set condition may include at least one of the following: providing a sperm measurement image measured by the personal sperm measurement apparatus 310 to the body fluid management server 100 (reward provided each time an image is provided), providing a measurement image to an external organization or company through the selection information provision unit 112, and enrolling in infertility-related insurance with an insurance company.

The insurance linkage unit 114 is linked with the body fluid state determination unit 104 and suggests insurance enrollment or insurance claim to a user when the sperm state changes from the care state to the normal state or from the care state to the abnormal state, or when the level of the care state decreases or increases to a set level, and upon receiving a positive response from the user, the insurance linkage unit 114 provides the insurance company with information on the user's sperm state determined by the body fluid state determination unit 104.

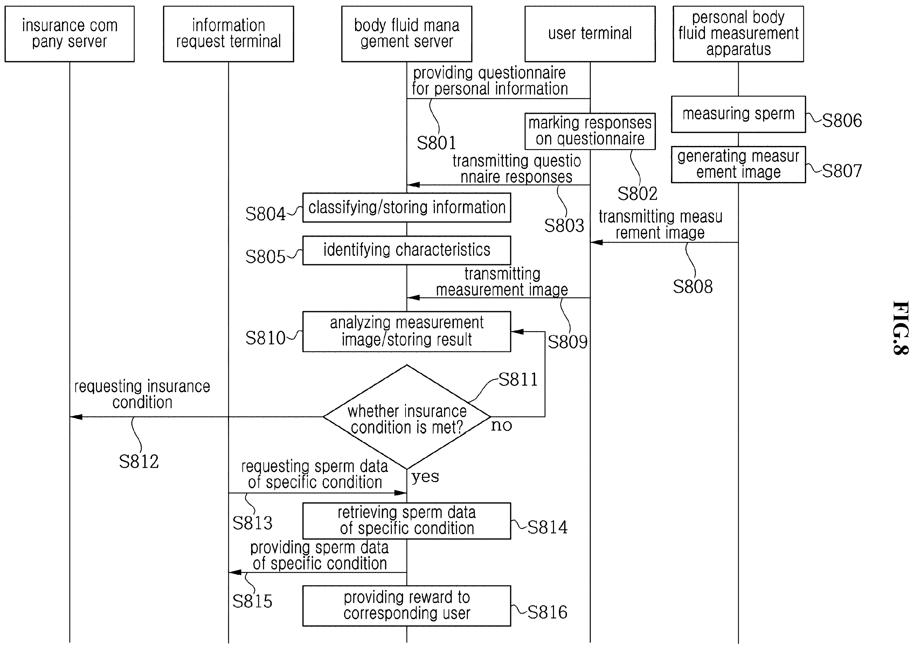

FIG. 8 is a data flowchart for the body fluid management method according to the third embodiment of the present disclosure, which is based on the operations of the body fluid management system according to the third embodiment of the present disclosure. The series of operations for generating and providing health management information based on measurement images, as shown in FIG. 8 is identical to that of the first or second embodiment, so detailed descriptions thereof are omitted.

Referring to FIG. 8, the body fluid management server 100 provides a questionnaire for personal information to a user terminal 320 to assess personal information (S801), and the user terminal 320 receives the user's response to the questionnaire to compile the responses (S803), then provides the questionnaire response to the body fluid management server 100 (S803)

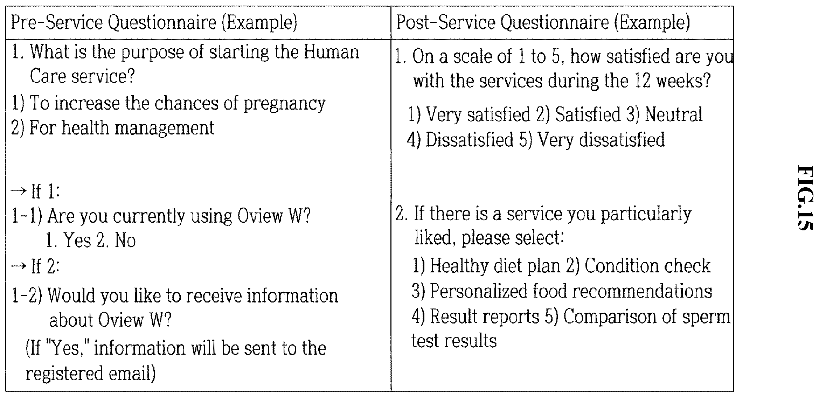

An example of the questionnaire provided from the body fluid management server 100 to the user terminal 320 is shown in FIG. 15.

The body fluid management server 100 receives the questionnaire responses to identify the user and classify and store the information by type (S804), and also assesses the user's age, location, and physical characteristics (including any illness), and then records the assessed information in the user information storage unit 106 (S805).

Meanwhile, when the user conducts sperm measurement using the personal sperm measurement apparatus 310, the personal sperm measurement apparatus 310 measures a sperm state (S806), generates a measurement image (S807), and provides the generated measurement image to the user terminal 320 (S808). The user terminal 320 receives the measurement image from the personal sperm measurement apparatus 310 and transmits the measurement image to the body fluid management server 100 as per the user's instruction (S809).

When receiving the measurement images from the user terminal 320, the body fluid management server 100 identifies the user, stores the received measurement image matched with the user, analyzes the measurement image measured by the personal sperm measurement apparatus 310, and assesses and stores state information of sperm (S810).

Based on the sperm state assessed in operation S810, the body fluid management server 100 determines whether an insurance condition is met (S811), and when the insurance condition is met, the body fluid management server 100 provides information on the insurance condition to an insurance company server to request or notify the user's insurance enrollment or claim (S812).

Meanwhile, when an external organization or company's information request terminal (or server) requests sperm data meeting a specific condition (S813), the body fluid management server 100 retrieves the sperm data meeting the specific condition from the measurement data storage unit 106 (S814) and provides the retrieved sperm data (measurement images) to the corresponding terminal (or server) (S815).

When the sperm data is provided to the external entity in this way, the body fluid management server 100 provides a reward to the user (S816).

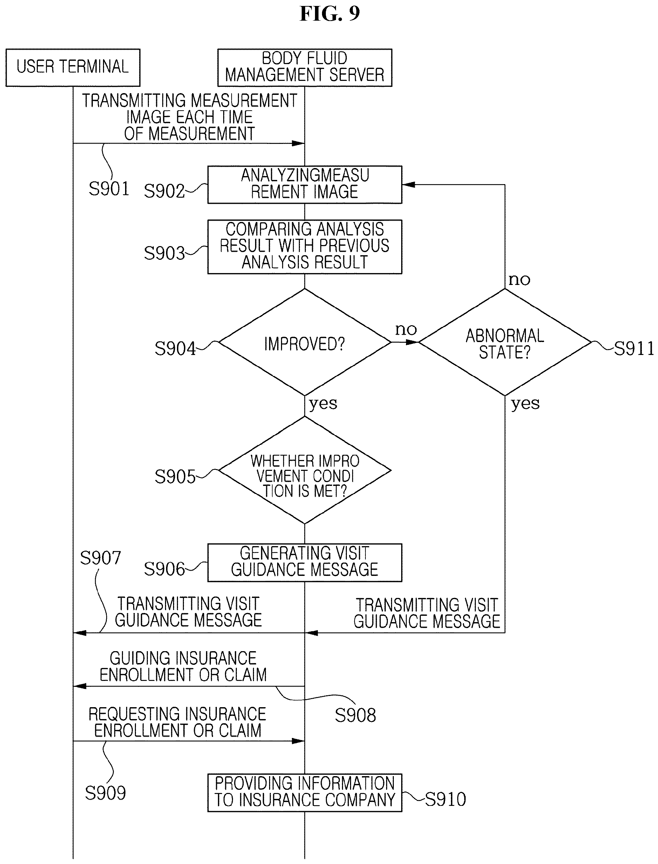

In the following, linking with the insurance company will be described in more detail with reference to FIG. 9. FIG. 9 is a data flowchart for an insurance company linking operation according to an embodiment of the present disclosure.

Referring to FIG. 9, whenever a user conducts sperm measurement using a personal sperm measurement apparatus 310, a user terminal 320 transmits a measurement image to a body fluid management server 100 (S901). Subsequently, the body fluid management server 100 analyzes the measurement image to assess state information of sperm (S902), and compares the assessed state information of sperm with a previous state (S903).

Through the comparison, the body fluid management server 100 determines whether the sperm has improved (S904), and if any improvement is detected, the body fluid management server 100 determines whether an improvement condition is met (S905). The improvement condition is a condition where the sperm's state has changed from a care state to a normal state or the level of the care state has increased to a set level.

When it is determined in S905 that the improvement condition for the sperm state is met, the body fluid management server 100 sends a visit guidance message to the user terminal 320 to advise the user to visit a hospital, along with an instruction indicating that the improvement condition is met (S907). Operation S907 aims to accurately measure the sperm's state.

In addition, the body fluid management server 100 assesses whether the user has been enrolled in infertility-related insurance based on the insurance information storage unit 111, and if subscribed, a message prompting insurance claim submission is sent, and if not, a message guiding enrollment to infertility-related insurance is sent (S908).

When receiving a response indicating intent to enroll in insurance or claim insurance premium in response to operation S908 from the user terminal 320 (S909), the body fluid management server 100 provides the user's current sperm state and state information to the insurance company, so that the user can enroll in insurance or claim insurance premium based on the current sperm state and state information (S910).

Conversely, when it is determined in operation S904 that the user's sperm state has not improved, the body fluid management server 100 determines whether the sperm state has deteriorated to an abnormal state (S911), and when the sperm state has deteriorated to the abnormal state, the body fluid management server 100 transmits a visit guidance message to the user terminal 320 (S907). Then, when the user has been enrolled in infertility-related insurance, a message prompting insurance claim submission is sent (S908), and upon a user's request, the body fluid management server 100 provides the user's sperm state and state information for insurance claim to the insurance company (S910).

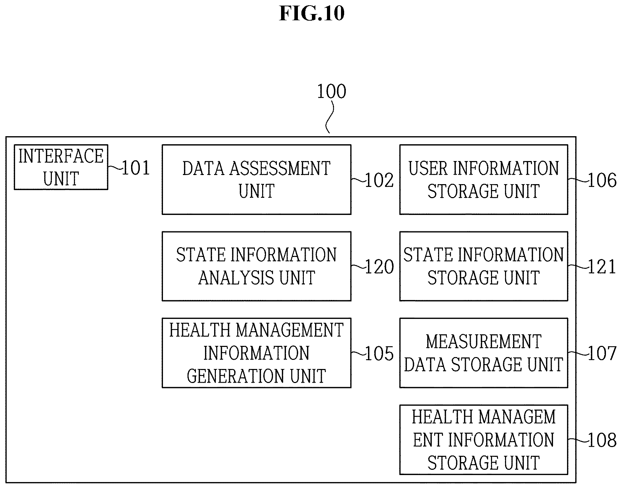

FIG. 10 is a block diagram of a body fluid management server according to a fourth embodiment of the present disclosure. Referring to FIG. 10, a body fluid management server according to the fourth embodiment of the present disclosure includes an interface unit 101, a data identification unit 102, a state information analysis unit 120, a health management information generation unit 105, a user information storage unit 106, a measurement data storage unit 107, a health management data storage unit 108, and a state information storage unit 121.

The interface unit 101 performs a communication interface with an external terminal connected from the outside to enable data transmission and reception with the external terminal.

The data identification unit 102 identifies the type of data received through the interface unit 101 and identifies the sender, i.e., the user, by assessing source information of the data. In addition, if the received data is personal sperm data measured by a personal sperm measurement apparatus 310, the data identification unit 120 transmits the personal sperm data to a clinic sperm measurement apparatus 200 or a clinic terminal.

The state information analysis unit 120 stores the user's clinic measurement images and the state information of the user's sperm (the number of sperm, the degree of sperm motility, the degree of sperm straightness, and the number of immature sperm) transmitted from the clinic sperm measurement apparatus 200 or a clinic terminal (not shown), and analyzes the state information of the user's sperm, transmitted from the clinic sperm measurement apparatus 200 or the clinic terminal (not shown) to assess the user's sperm state (normal state, abnormal state, care state, and level). While named to be distinguished from the functions of the body fluid management server 100 in the first to third embodiments, the state information analysis unit 120 performs the same functions as the body fluid state determination unit 104, and is thus referred to as the body fluid state determination unit 104 in the following claims.

The health management information generation unit 105 utilizes an analysis result from the state information analysis unit 120, generates health management information (i.e., sperm enhancement program) for a user whose sperm state is a care state, and, at this point, generates personalized health management information based on a level of the care state. For example, higher levels of the care state involve providing management information that emphasizes increased exercise intensity and duration for the user, along with recommending foods known to enhance sperm quality. And, when receiving personal sperm data from the user after providing the health management information to the user, the health management information generation unit 105 may provide new or one-time health management information depending on the state of the received personal sperm data.

The user information storage unit 106 stores personal information (e.g., name, age, residence, weight, height, etc.) for registered users. The measurement data storage unit 107 stores each user's clinic sperm data and personal sperm data. The health management information storage unit 108 stores health management information provided for each user.

The measurement data storage unit 107 stores each user's clinic sperm data and personal sperm data. The health management information storage unit 108 stores health management information provided for each user.

The state information storage unit 121 stores the state information of the user's sperm, transmitted from the clinic sperm measurement apparatus 200 or a clinic terminal.

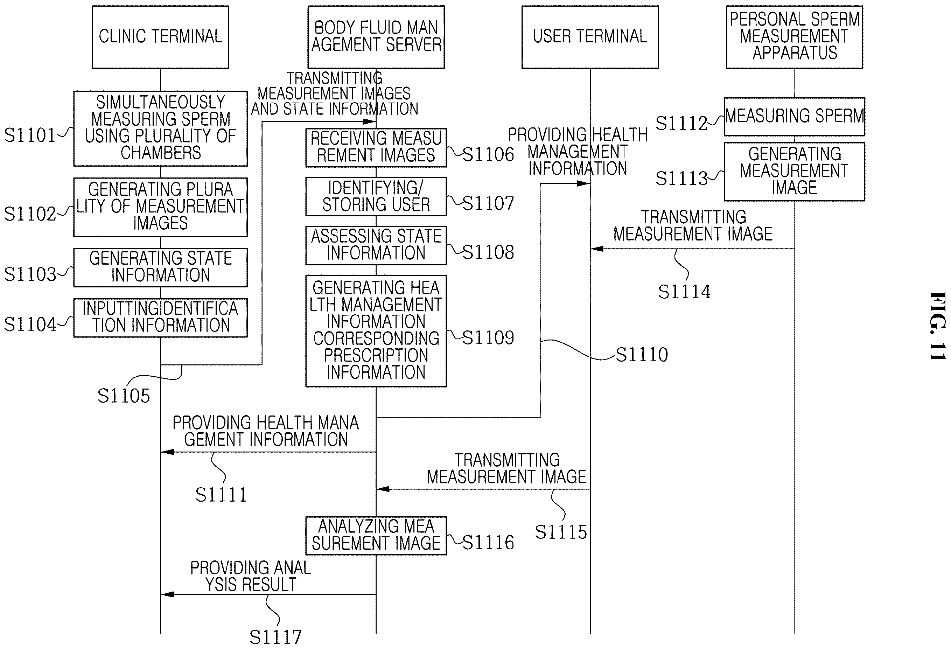

FIG. 11 is a data flowchart for a body fluid management method according to the fourth embodiment of the present disclosure, and a clinic terminal includes a clinic sperm measurement apparatus 200 and a clinic terminal connected to the sperm measurement apparatus 200 over a network.

Referring to FIG. 11, when a user visits a clinic, the measurement is initiated with the user's sperm placed into a plurality of chambers of the clinic sperm measurement apparatus 200. Accordingly, the clinic sperm measurement apparatus 200 simultaneously measures the user's sperm placed in the plurality of chambers (S1101), generates a plurality of measurement images for the sperm in the respective chambers, and provides the plurality of measurement images to a clinic terminal (S1102). The clinic terminal generates sperm state information and incorporates the sperm state information into the plurality of measurement images according to an instruction from a clinic administrator(S1103), add the user's identification information (e.g., name or corresponding code number) in the plurality of measurement images (S1104), and then transmits a measurement image message, containing the plurality of measurement images, the sperm state information, and the user's information, to the body fluid management server 100 (S1105).

The body fluid management server 100 receives the measurement image message (S1106), and identifies the user based on the user's identification information included in the message and stores the plurality of measurement images matched with the user in the measurement data storage 106 (S1107).

In addition, the body fluid management server 100 assesses the number of sperm, sperm activity, sperm straightness, and sperm deformity based on the state information of the user's sperm included in the measurement image message (S1108). Then, the body fluid management server 100 assesses the user's final sperm state information using the plurality of assessed states of sperm (S1108).

The final state information indicates whether the user's sperm is in a normal, abnormal, or care state, whether medicine prescription is possible, and what level of the care state if the user's sperm is in the care state.

When the sperm state is normal or abnormal, the body fluid management server 100 notifies the clinic sperm measurement apparatus 200 and the user terminal 320 of the state and stops operation. On the other hand, the body fluid management server 100 generates health management information corresponding to an analysis result (S1109), provides the generated health management information to the user terminal 320 and the clinic sperm measurement apparatus 200 to inform both the user and a clinic administrator (S1110, S1111). Of course, even if the user's sperm is in the care state, the body fluid management server 100 also notifies the user terminal 320 and the clinic sperm measurement apparatus 200 of the care state.

When the user checks the health management information through the user terminal 320, the user follows the schedule included in the health management information to improve the sperm state by consuming specific foods or performing exercises. Next, the user measures his sperm state using the personal sperm measurement apparatus 310 in order to assess the impact of the user's actions in accordance with the health management information.

Accordingly, the personal sperm measurement apparatus 310 measures a state of sperm (S1112), generates a measurement image (S1113), and provides the generated measurement image to the user terminal 320 (S1114).

The user terminal 320 receives the measurement image from the personal sperm measurement apparatus 310, displays the received measurement image on a screen of the user terminal 320 through a service application for user's verification, and then transmits the measurement image to the body fluid management server 100 as per the user's instruction (S1115).

When receiving the measurement image from the user terminal 320, the body fluid management server 100 identifies the user, stores the received measurement image matched with the user, and then analyzes the measurement image measured by the personal sperm measurement apparatus 310 to assess state information of sperm (S1116).

The body fluid management server 100 provides the state information of the user's sperm assessed in operation S318 to the clinic measurement apparatus 200 (S1117). Of course, the body fluid management server 100 may provide the user terminal 310 with the state information of the user's sperm assessed in operation S318.

Therefore, a clinic administrator is able to monitor whether the user is following the health management information by receiving and verifying the user's sperm state, measured through the personal sperm measurement apparatus 310, via the body fluid management server 100.

Meanwhile, the body fluid management system according to the fourth embodiment of the present disclosure may be modified in various ways. For example, the body fluid management system according to the fourth embodiment may include the blockchain unit 109 which is part of the configuration of the body fluid management system according to the second embodiment of the present disclosure, or may further include at least one of the personal information assessment unit 110, the selection information provision unit 112, the reward provision unit 113, and the insurance linkage unit 114, which are part of the configuration of the body fluid management system according to the third embodiment of the present disclosure.

In addition, the body fluid management system according to the fourth embodiment of the present disclosure may also vary depending on the various modifications to the body fluid management system according to the fourth embodiment of the present disclosure.

Meanwhile, in the above-described embodiment, it has been described that the clinic measurement apparatus 200 receives information transmitted directly from the body fluid management server 100: however, if the clinic measurement apparatus 200 lacks the capability to directly receive information from the body fluid management server 100, the information transmitted from the body fluid management server 100 is received by a clinic administrator's terminal (e.g., smartphone or computer) or a clinic terminal (e.g., computer or server) installed in the clinic.

In the above-described embodiment, the body fluid analysis unit 103, the body fluid state determination unit 104, the health management information generation unit 105, the blockchain unit 109, the personal information assessment unit 110, and the selection information provision unit 112, the reward provision unit 113, and the insurance linkage unit 114 perform distinct functional operations by a data processing processor of the body fluid management server 100, while each storage unit 106, 107, 108, and 111 corresponds to a memory of the body fluid management server 100 and the interface unit 101 corresponds to a communication module of the body fluid management server 100.

The technical features disclosed in each embodiment of the present disclosure are not limited to a corresponding embodiment, and unless incompatible with each other, the technical features disclosed in each embodiment may be applied in combination to other embodiments.

Therefore, although each embodiment is described mainly about an individual technical feature, the technical features of the embodiments of the present disclosure may be applied in combination, unless incompatible with each other.

The present disclosure is not limited to the above-described embodiments and the accompanying drawings, and various modifications and changes may be made in view of a person skilled in the art to which the present disclosure pertains. Therefore, the scope of the present disclosure should be determined by the scope of the appended claims, and equivalents thereof.

## Claims

1. A body fluid management server comprising:
an interface unit configured to perform communication interface with a clinic terminal in communication with a clinic sperm measurement apparatus and a user terminal in communication with a personal sperm measurement apparatus;
a body fluid analysis unit configured to assess state information of sperm by analyzing clinic sperm data and personal sperm data received via the interface unit, and provide state information regarding the personal sperm data to the user terminal and the clinic terminal for monitoring by the user and a clinic administrator;
a body fluid state determination unit configured to determine a final state of the sperm of the user based on the state information of the sperm assessed by the body fluid analysis unit;
a health management information generation unit configured to generate health management information suitable for the user in accordance with a level of a care state from among the final state determined by the body fluid state determination unit;
a measurement data storage unit configured to store each user's clinic sperm data and personal sperm data; and
a health management information storage unit configured to store the health management information provided to each user.

2. The body fluid management server of claim 1, wherein the body fluid analysis unit analyzes each of a plurality of measurement images included in the clinic sperm data to assess state information of sperm in the respective measurement images, and assesses final state information by averaging the determined state information of sperm in the respective measurement images.

3. The body fluid management server of claim 1, wherein when the interface unit receives biometric information of the user from the user terminal, the body fluid state determination unit determines a final state based on the sperm state information assessed by the body fluid analysis unit and the biometric information of the user.

4. The body fluid management server of claim 1, further comprising:
a blockchain unit configured to generate a block by encrypting the clinic sperm data and the personal sperm data and transmit the generated block to each blockchain node of a blockchain associated with the user to share information.

5. The body fluid management server of claim 1, further comprising:
a user information assessment unit configured to inquire the user about personal information regarding at least one of an existing disease, a dietary habit, an alcohol consumption status, and a smoking status, receive a response from the user, and assess the user's characteristics based on the received personal information; and
a selection information provision unit configured to, in response to receiving a request from an external organization or company for sperm data exhibiting a specific individual's characteristics, retrieve the data exhibiting the specific individual's characteristics based on the characteristics assessed by the user information assessment unit and provide the retrieved data to the external organization or company.

6. The body fluid management server of claim 5, further comprising:
a reward provision unit configured to provide a reward to a user who has provided the personal sperm data or a user who owns the sperm data provided to the external organization or company through the selective information provision unit.

7. The body fluid management server of claim 5, further comprising:
an insurance linkage unit configured to suggest insurance enrollment or claim to a user when the sperm state determined by the body fluid state determination unit has changed from the care state to the normal state, from the care state to the abnormal state, or when a level of the care state decreases or increases to a set level, and provide an insurance company with information on the user's sperm state when receiving a positive response from the user.

8. A body fluid management server comprising:
an interface unit configured to perform communication interface with a clinic terminal in communication with a clinic sperm measurement apparatus and a user terminal in communication with a personal sperm measurement apparatus;
a data identification unit configured to identify a type of data received through the interface unit and a user for the data, and provide the data to the clinic terminal when the received data is personal sperm data;
a body fluid state determination unit configured to store the clinic sperm data and personal sperm data received through the interface unit, and analyze state information of sperm received from the clinic terminal through the interface unit to assess a final sperm state of a user;
a health management information generation unit configured to provide a user whose sperm is in a care state with health management information based on an analysis result from the state information analysis unit;
a measurement data storage unit configured to store each user's clinic sperm data and personal sperm data;
a health management information storage unit configured to store health management information provided to each user; and
a state information storage unit configured to store the state information of sperm, transmitted from the clinic terminal.

9. A health management method performed through a body fluid management server which is connected to a user terminal in communication with a personal sperm measurement apparatus and a clinic terminal in communication with a clinic sperm measurement apparatus to manage a health status of sperm, the method comprising:
analyzing, by the body fluid management server, received clinic sperm data to assess first state information of sperm;
assessing, by the body fluid management server, a final state of a user's sperm based on the first state information;
providing, by the body fluid management server, health management information corresponding to the final state to the user terminal and the clinic terminal;
analyzing, by the body fluid management server, received personal sperm data to assess second state information of the sperm; and
providing, by the body fluid management server, the second state information to the user terminal and the clinic terminal so that the user and a clinic administrator monitor the second state information.

10. The health management method of claim 9, wherein the assessing of the first state information comprises analyzing each of a plurality of measurement images included in the clinic sperm data to assess state information of sperm in the respective measurement images, and assessing the first state information by averaging the assessed state information of sperm in the respective measurement images.

11. The health management method of claim 9, wherein the assessing of the final state is determining the final state based on the first state information and biometric information of the user when the biometric information of the user is received from the user terminal.

12. The health management method of claim 9, further comprising:
generating a block by encrypting the clinic sperm data and the personal sperm data and transmitting the generated block to each blockchain node of a blockchain associated with the user to share information.

13. The health management method of claim 9, further comprising:
inquiring the user about personal information regarding at least one of an existing disease, a dietary habit, an alcohol consumption status, and a smoking status, receiving a response from the user, and assessing the user's characteristics based on received personal information; and
in response to receiving a request from an external organization or company for sperm data exhibiting a specific individual's characteristics, retrieving the data exhibiting the specific individual's characteristics and providing the retrieved data to the external organization or company.

14. The health management method of claim 13, further comprising:
providing a reward to a user who has provided the personal sperm data or a user owns the sperm data provided to the external organization or company.

15. The health management method of claim 9, further comprising:
suggesting insurance enrollment or insurance claim to a user when the sperm state determined by the body fluid state determination unit has changed from a care state to a normal state, from the care state to an abnormal state, or when a level of the care state decreases or increases to a set level, and providing an insurance company with information on the user's sperm state when receiving a positive response from the user.

16. A health management method performed through a body fluid management server which is connected to a user terminal in communication with a personal sperm measurement apparatus and a clinic terminal in communication with a clinic sperm measurement apparatus to manage health state of sperm, the method comprising:
storing, by the body fluid management server, received clinic sperm data or received personal sperm data;
analyzing, by the body fluid management server, sperm state information regarding the received clinic sperm data to assess the final state of a user's sperm;
providing, by the body fluid management server, health management information corresponding to the final state to the user terminal and the clinic terminal; and
in response to receiving personal sperm data, providing, by the body fluid management server, the personal sperm data to the clinic terminal so that clinic administrator monitor the personal sperm data.
